# EUROPEAN PATENT SPECIFICATION

(11) **EP 4 489 672 B1**
(45) Date of publication and mention of the grant of the patent: **08.04.2026**
(21) Application number: 23708914.9
(22) Date of filing: 24.02.2023
(51) Int. Cl.: A61B 34/10

(54) **METHOD FOR CALCULATING THE SHAPE OF A ROD OF A PEDICLE SCREW SYSTEM FOR SPINAL FUSION**
VERFAHREN ZUR BERECHNUNG DER FORM EINER STANGE EINES PEDIKELSCHRAUBENSYSTEMS ZUR WIRBELSÄULENFUSION
PROCÉDÉ DE CALCUL DE LA FORME D'UNE TIGE D'UN SYSTÈME DE VIS PÉDICULAIRES POUR SPONDYLODÈSE

(30) Priority: 08.03.2022 IT 202200004382
(43) Date of publication of application: 15.01.2025
(73) Proprietor: Medacta International SA, 6874 Castel San Pietro (CH)
(72) Inventor: SICCARDI, Francesco, 6874 CASTEL SAN PIETRO (CH); FIECHTER, Meinrad, 6874 CASTEL SAN PIETRO (CH)
(74) Representative: Grassi, Stefano
(86) International application number: PCT/IB2023/051721
(87) International publication number: WO 2023/170505

(56) References cited:
- WO-A1-2018/131045
- WO-A1-2020/201353
- US-A1- 2016 242 857
- US-A1- 2018 092 699
- US-A1- 2018 301 213
- US-A1- 2020 015 857
- US-A1- 2020 345 420
- US-B1- 9 968 408

## Description

The present invention relates to a method for calculating the shape of a rod of a pedicle screw system for spinal fusion.

Spinal fusion is a surgery treatment to permanently connect two or more vertebrae of a spine, thus eliminating motion between them for stabilizing a vertebral segment of a patient.

Rods are usually used to connect pedicle screws which are inserted into vertebral bodies, to strengthen the spine of a patient.

Pedicle screws are surgical instruments which allow to reinforce spinal fusion in cases of injury of the spine of a patient and they comprise a tulip and a screw.

Curvature abnormality of the spine (scoliosis or kyphosis), spinal vertebrae injury, protrusion of the cushioning disk between vertebrae (slipped disk, herniated nucleus pulposus) and weak or unstable spine caused by diseases are some cases in which spinal fusion is usually applied.

The pedicle screw is a particular type of bone screw designed for implantation into a vertebral pedicle that provides means for grasping a spinal segment at any level of a spinal column (cervical, thoracic and lumbosacral).

A rod is a metal cylinder implant used in spinal surgery to stabilize a vertebral segment, by connecting pedicle screws inserted into adjacent vertebral bodies in order to prevent motion and allow fusion to occur across the disc space.

Usually, rods are bended inside the operating room during surgery, depending on the screw position and the correction required for each patient.

The rod bending process requires a lot of assumption since the rod has to go into the tulip of the pedicle screws, but it is also used as point of contact in order to correct the spine.

In addition to that, it is extremely difficult to bend the rod freehand in the correct way, during surgical operation, by considering different parameters like the optimal curvature to apply to the rod to achieve a desired correction.

It would therefore be desirable that the patient could receive a rod with an optimal curvature, while reducing the surgical operating time. A patient specific rod or a rod template could therefore provide a lot of value to the patient and to the surgeon.

Example of devices and methods operating in this technical field can be found in documents: US2018/301213A1, US2018/092699A1 and US2020/345420A1 .

There is therefore the need to have a pre-operative planning method for the creation of a patient specific rod, thus overcoming the problems of the prior art.

These and other objects are fully achieved by virtue of a method for calculating the shape of a rod of a pedicle screw system for spinal fusion having the characteristics defined in independent claim 1, and by a system for calculating the shape of a rod of a pedicle screw system for spinal fusion having the characteristics defined in claim 9.

Preferred embodiments of the invention are specified in the dependent claims, whose subject-matter is to be understood as forming integral or integrating part of the present description.

Further characteristic and advantages of the present invention will become apparent from the following description, provided merely by way of non-limiting example, with reference to the attached drawings, in which:
- Figure 1 shows a block diagram of the step of a method for calculating the shape of a rod of a pedicle screw system for spinal fusion according to the present invention; and
- Figure 2 shows a system for calculating the shape of a rod of a pedicle screw system for spinal fusion according to the present invention.

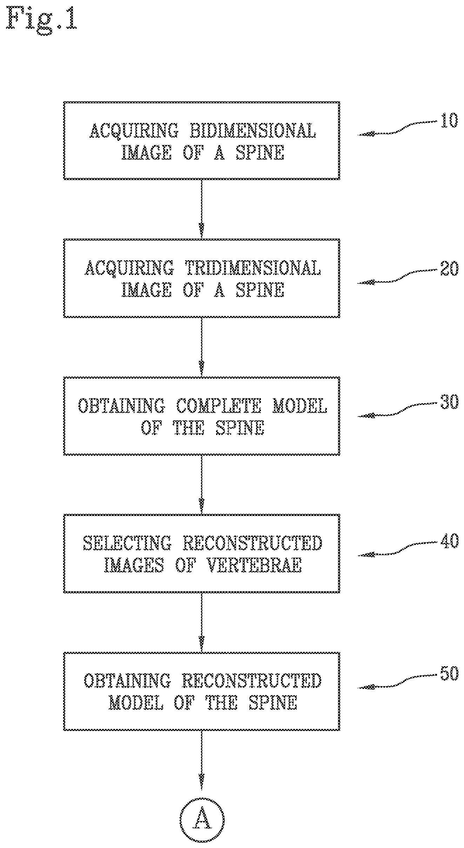

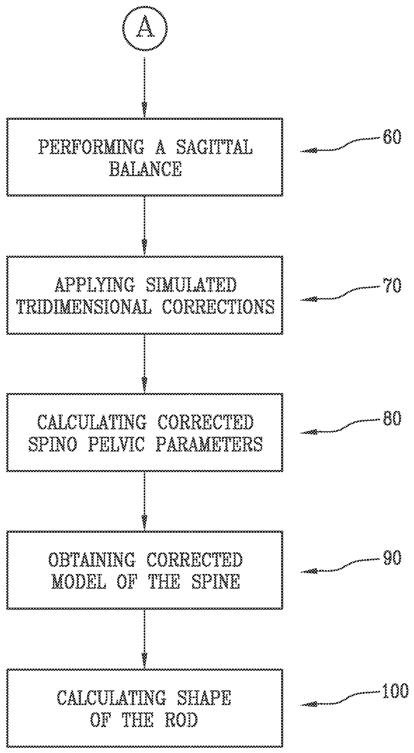

Figure 1 shows a block diagram of the step of a method for calculating the shape of a rod of a pedicle screw system for spinal fusion according to the present invention.

In a first step 10 at least one bidimensional x-ray image of the spine of a patient is acquired, preferably in standing position, preferably a lateral x-ray (sagittal) image or an anterior-posterior x-ray image, the bidimensional image preferably including a pelvic region such as the sacrum and the hip joint-portion the thoracolumbar spine as well as the C7 in the cervical spine. The bidimensional image includes a plurality of vertebrae.

In a second step 20, a tridimensional scan image of the spine of the patient is acquired, for example a CT or a MRI image. Subsequently, a segmentation and a tridimensional reconstruction of the vertebrae contained in such tridimensional scan image is performed, for each level, as here below disclosed.

As an alternative to the tridimensional scan acquisition, it is possible to acquire all the tridimensional images of the vertebrae from a predefined database or to acquire partial image of the patient from the tridimensional scan acquisition and partial image of the spine from the database, as here below detailed.

In a further step 30, a tridimensional virtual complete model of the spine is obtained, in a manner per se known, starting from the scan image of the spine of step 20 and merged with the information provided from the x-ray acquisition of step 10. Advantageously, tridimensional images of a lower extremity of the spine (sacrum and/or pelvis) are added to the complete model of the spine.

As above indicated, if at step 20 the tridimensional scan image of the spine cannot be completely acquired or cannot be acquired for certain levels of the spine, in a step 40, a selection of at least one tridimensional reconstructed vertebra/sacrum/pelvic image from a predefined database of patient specific tridimensional vertebrae is performed, wherein the reconstructed image is selected based on a best fit between the tridimensional (3D) geometry of the vertebrae of the database and the contour given for each vertebra by the bidimensional x-ray image, i.e. the sagittal or anterior-posterior image of step 10, and then they are combined, at step 50, to obtain a reconstructed tridimensional model of the spine.

In particular, at step 50, the at least one tridimensional image of the reconstructed vertebra of the database is matched, for example by using a DDR (Digital Reconstructed Radiograph) algorithm or any other algorithm using particular landmarks or geometries to do a matching of tridimensional information, to the bidimensional images of the vertebrae of the x-ray acquisition, in order to obtain the tridimensional virtual reconstructed model of the spine and the pelvic region.

At this point, at step 60, either the x-ray image or the tridimensional model of the spine (complete or reconstructed) is used to perform a first sagittal balance analysis to calculate spino pelvic parameters such as pelvic incident, sacral slope, pelvic tilt, lumbar lordosis, thoracic kyphosis, sagittal vertical axis, and then these spino pelvic parameters are compared with predetermined range values reported in literature.

In negative case, i.e., the spino pelvic parameters are not within the predetermined range values, at step 70, predetermined tridimensional simulated corrections are applied to the tridimensional model of the spine, e.g. an open or closed wedge.

At step 80, corrected spino pelvic parameters are calculated on such tridimensional model of the spine corrected with the simulated corrections and compared with the predetermined range values reported in literature to understand if a desired balanced spine is achieved.

In negative case of the check of step 80, step 70 is repeated until corrected spino pelvic parameters belonging to the predetermined range values are obtained.

Alternatively, if only a subset of the corrected spino pelvic parameters are within the range values, the method may proceed to the next step.

In addition to that, the bidimensional x-ray image and/or a coronal plane of the tridimensional model of the spine are elaborated to determine, in a manner per se known, the Cobb angle and the correctied parameters if necessary.

In an alternative embodiment of the invention, first predetermined additional correction factors are added to modify the corrected spino pelvic parameters, based on the surgical technique to apply as well as the expected mobility and stiffness on the spine among others, to take plastic and elastic material deformation of the rod into account.

In a further alternative embodiment of the invention, second predetermined additional correction factors are applied to the corrected spino pelvic parameters to take into account pelvic and lower extremity alignment such as, for example, mechanical axis, gait and general movement analysis.

The corrected spino pelvic parameters as above determined correspond to acceptable spino pelvic parameters and, if necessary, to an adequate coronal plane correction (Cobb angle).

The corrected spino pelvic parameters have been obtained, as above disclosed, by applying the predetermined tridimensional simulated corrections to the tridimensional model of the spine, these simulated corrections corresponding, in a manner per se known, to predetermined surgical steps that have to be applied to the patient by the surgeon, for example, the insertion of cages in predetermined points of the spine, to get the balanced spine, as here below detailed.

When the corrected spino pelvic parameters as well as the Cobb angle are within the predetermined range values, in a subsequent step 90, a tridimensional corrected model of the spine is obtained based on said tridimensional simulated corrections.

Such tridimensional simulated corrections include insertion, in predetermined positions, of virtual screws and/or, advantageously, virtual cages, into the tridimensional model of the spine.

Screw entry points and entry directions of the virtual screws are calculated in a manner per se known. Virtual screws and screw trajectories can be applied either to the right and/or to the left side of the spine in particular the pedidle, the sacrum and the Ilium.

Advantageously, it is possible to calculate the entry point of the screws or calculate the entry points of the screws and the position of the virtual template of the screws.

If all the spino pelvic parameters and the Cobb angle are within the predetermined range values, in a further step 100 the shape of the rod is calculated, based on the position of the virtual screws or the defined screw entry points, by calculating the mathematical function which best approximates the curve passing through the screw insertion points (i.e, through the virtual screws themselves or through their entry points).

The resulting mathematical formula represents the shape of the rod. Additional parameters for under/overcorrection of the rod can be implemented depending on e.g., an expected rigidity of the spine or a particular technique used for the correction

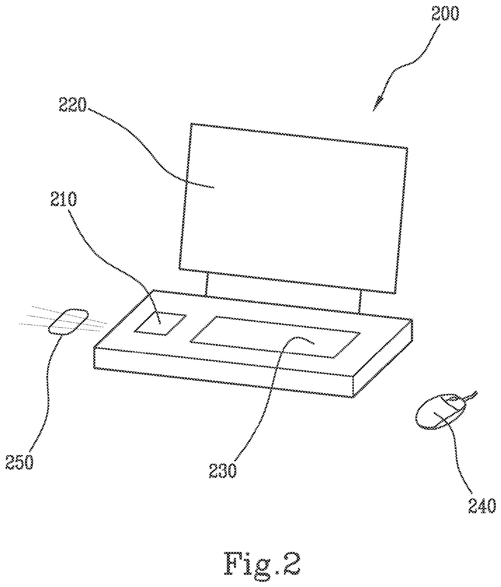

The process according to the invention is performed by a system of the type illustrated in Figure 2 comprising a workstation 200 of known type having a processing subsystem 210, a display device 220, a keyboard 230, a pointing device (mouse) 240, and a device for connecting to a local area network (network bus) 250.

Alternatively, the processing system may be of a distributed type (not illustrated) having a processing subsystem and local or remote peripheral input/output devices.

The workstation 200 or distributed system is arranged to process processing groups or modules and computational programs that are stored on a disk or are accessible over a network and that are suitable for displaying the described process to display results on the device 220.

The described solutions are considered to be well known to one skilled in the art and will therefore not be described herein as they are not relevant to the implementation purposes and understanding of the invention.

Clearly, the principle of the invention remaining the same, the embodiments and the details of production can be varied considerably from what has been described and illustrated purely by way of non-limiting example, without departing from the scope of protection of the present as defined in the attached claims.

## Claims

1. Method for calculating a shape of a rod of a pedicle screw system for spinal fusion comprising the steps of:
a) acquiring (10) at least one bidimensional image of the spine of a patient;
b)selecting (40) at least one tridimensional reconstructed image of a vertebra from a predefined database, said reconstructed image having a predetermined match with vertebrae images of the bidimensional image;
c) obtaining (30) a tridimensional model of the spine combining (50) the reconstructed image and the bidimensional image to obtain a reconstructed tridimensional model of the spine;
d) performing (60) a sagittal balance analysis of the bidimensional image or the tridimensional model to calculate spino pelvic parameters and comparing said spino pelvic parameters with predetermined range values;
e) applying (70) tridimensional simulated corrections to the tridimensional model of the spine;
f) calculating (80) corrected spino pelvic parameters on the tridimensional model of the spine corrected with the simulated corrections and checking if at least a subset of the corrected spino pelvic parameters are within the predetermined range values,
g) in negative case, repeating steps e) - f);
i) in positive case, obtaining (90) a tridimensional corrected model of the spine based on such tridimensional simulated corrections, said tridimensional simulated corrections including insertion of a plurality of virtual screws into the tridimensional model of the spine;
j) calculating (100) the shape of the rod as the mathematical function which best approximates a curve passing through the plurality of virtual screws of the tridimensional corrected model of the corrected spine.

2. Method for calculating the shape of a rod according to claim 1, wherein the step of combining (50) the at least one reconstructed image and the bidimensional image comprises applying a Digital Reconstructed Radiograph, DDR, algorithm.

3. Method for calculating the shape of a rod according to claims 1 or 2, wherein the at least one bidimensional image comprises a lateral and/or an anterior-posterior x-ray image.

4. Method for calculating the shape of a rod according to any of the preceding claims, wherein the step of selecting (40) is performed by selecting (40) at least one tridimensional reconstructed image of each vertebra from a predefined database or the method comprises a step of acquiring (20) a tridimensional image of at least one vertebra of the spine of the patient and the step of obtaining (30) a tridimensional model of the spine is performed by combining for each vertebra the bidimensional image with the reconstructed image or the tridimensional image.

5. Method for calculating the shape of a rod according to claim 4, wherein the tridimensional image of the spine comprises a MRI or CT image.

6. Method for calculating the shape of a rod according to any of the preceding claims, wherein the spino pelvic parameters comprise at least one among: pelvic incident, sacral slope, pelvic tilt, lumbar lordosis, thoracic kyphosis, sagittal vertical axis.

7. Method for calculating the shape of a rod according to any of the preceding claims, further comprising adding first predetermined correction factors to the corrected spino pelvic parameters, based on surgical technique to apply and/or expected mobility and stiffness on the spine, thus taking into account plastic and elastic material deformation of the rod.

8. Method for calculating the shape of a rod according to claim 7, further comprising applying second predetermined correction factors to the corrected spino pelvic parameters to take into account pelvic and lower extremity alignment.

9. System for calculating the shape of a rod of a pedicle screw system for spinal fusion comprising a workstation (200) having a processing subsystem (210) arranged to:
a) acquiring (10) at least one bidimensional image of the spine of a patient;
b) selecting (40) at least one tridimensional reconstructed image of a vertebra from a predefined database, said reconstructed image having a predetermined match with vertebrae images of the bidimensional image;
c) obtaining (30) a tridimensional model of the spine combining (50) the reconstructed image and the bidimensional image to obtain a reconstructed tridimensional model of the spine;
d) performing (60) a sagittal balance analysis of the bidimensional image or the tridimensional model to calculate spino pelvic parameters and comparing said spino pelvic parameters with predetermined range values;
e) applying (70) tridimensional simulated corrections to the tridimensional model of the spine;
f) calculating (80) corrected spino pelvic parameters on the tridimensional model of the spine corrected with the simulated corrections and checking if the corrected spino pelvic parameters are within the predetermined range values,
g) in negative case, repeating steps e) - f);
i) in positive case, obtaining (90) a tridimensional corrected model of the spine based on such tridimensional simulated corrections, said tridimensional simulated corrections including insertion of a plurality of virtual screws into the tridimensional model of the spine;
j) calculating (100) the shape of the rod as the mathematical function which best approximates a curve passing through the plurality of virtual screws of the tridimensional corrected model of the corrected spine.

## Patentansprüche

1. Verfahren zur Berechnung der Form einer Stange eines Pedikelschraubensystems zur Wirbelsäulenfusion, das die folgenden Schritte umfasst:
a) Erfassen (10) mindestens eines zweidimensionalen Bildes der Wirbelsäule eines Patienten;
b) Auswählen (40) mindestens eines dreidimensionalen rekonstruierten Bildes eines Wirbels aus einer vordefinierten Datenbank, wobei das genannte rekonstruierte Bild eine vorgegebene Übereinstimmung mit Wirbelbildern des zweidimensionalen Bildes besitzt;
c) Erhalten (30) eines dreidimensionalen Modells der Wirbelsäule durch Kombinieren (50) des rekonstruierten Bildes und des zweidimensionalen Bildes, um ein rekonstruiertes dreidimensionales Modell der Wirbelsäule zu erhalten;
d) Durchführen (60) einer sagittalen Gleichgewichtsanalyse des zweidimensionalen Bildes oder des dreidimensionalen Modells zur Berechnung von Wirbelsäulen-Becken-Parametern und Vergleichen der genannten Wirbelsäulen-Becken-Parameter mit vorgegebenen Bereichswerten;
e) Anbringen (70) dreidimensionaler simulierter Korrekturen an dem dreidimensionalen Modell der Wirbelsäule;
f) Berechnen (80) korrigierter Wirbelsäulen-Becken-Parameter auf dem dreidimensionalen Modell der Wirbelsäule, das mit den simulierten Korrekturen korrigiert wurde, und Überprüfen, ob mindestens eine Teilgruppe der korrigierten Wirbelsäulen-Becken-Parameter innerhalb der vorgegebenen Bereichswerte liegt,
g) im negativen Fall, Wiederholen der Schritte e) - f);
i) im positiven Fall, Erhalten (90) eines dreidimensionalen korrigierten Modells der Wirbelsäule basierend auf solchen dreidimensionalen simulierten Korrekturen, wobei die genannten dreidimensionalen simulierten Korrekturen das Einsetzen einer Vielzahl von virtuellen Schrauben in das dreidimensionale Modell der Wirbelsäule einschließen;
j) Berechnen (100) der Form der Stange als die mathematische Funktion, die sich am besten einer Kurve annähert, die durch die Vielzahl der virtuellen Schrauben des dreidimensionalen korrigierten Modells der korrigierten Wirbelsäule verläuft.

2. Verfahren zur Berechnung der Form einer Stange nach Anspruch 1, wobei der Schritt des Kombinierens (50) des mindestens einen rekonstruierten Bildes und des zweidimensionalen Bildes das Anwenden eines Algorithmus für ein digitales rekonstruiertes Röntgenbild, DRR, umfasst.

3. Verfahren zur Berechnung der Form einer Stange nach Anspruch 1 oder 2, wobei das mindestens eine zweidimensionale Bild ein laterales und/oder ein anteriorposteriores Röntgenbild umfasst.

4. Verfahren zur Berechnung der Form einer Stange nach einem beliebigen der vorangegangenen Ansprüche, wobei der Schritt des Auswählen (40) durch Auswählen (40) mindestens eines dreidimensionalen rekonstruierten Bildes jedes Wirbels aus einer vordefinierten Datenbank durchgeführt wird oder das Verfahren einen Schritt des Erfassens (20) eines dreidimensionalen Bildes mindestens eines Wirbels der Wirbelsäule des Patienten umfasst, und der Schritt des Erhaltens (30) eines dreidimensionalen Modells der Wirbelsäule durch Kombinieren des zweidimensionalen Bildes mit dem rekonstruierten Bild oder dem dreidimensionalen Bild für jeden Wirbel durchgeführt wird.

5. Verfahren zur Berechnung der Form einer Stange nach Anspruch 4, wobei das dreidimensionale Bild der Wirbelsäule ein MRT- oder CT-Bild umfasst.

6. Verfahren zur Berechnung der Form einer Stange nach einem beliebigen der vorangegangenen Ansprüche, wobei die Wirbelsäulen-Becken-Parameter mindestens einen der folgenden Parameter umfassen: Beckenvorfall, Sakralneigung, Beckenkippung, Lendenlordose, Brustkyphose, sagittale Vertikalachse.

7. Verfahren zur Berechnung der Form einer Stange nach einem beliebigen der vorangegangenen Ansprüche, weiter umfassend das Hinzufügen erster vorbestimmter Korrekturfaktoren zu den korrigierten Wirbelsäulen-Becken-Parametern, die auf der anzuwendenden Operationstechnik und/oder der erwarteten Mobilität und Steifigkeit der Wirbelsäule beruhen, wodurch die plastische und elastische Materialverformung der Stange berücksichtigt wird.

8. Verfahren zur Berechnung der Form einer Stange nach Anspruch 7, weiter umfassend die Anwendung zweiter vorbestimmter Korrekturfaktoren auf die korrigierten Wirbelsäulen-Becken-Parameter, um die Ausrichtung des Beckens und der unteren Extremitäten zu berücksichtigen.

9. System zur Berechnung der Form einer Stange eines Pedikelschraubensystems zur Wirbelsäulenfusion, das eine Arbeitsstation (200) mit einem Verarbeitungssubsystem (210) umfasst, das zu Folgendem eingerichtet ist:
a) Erfassen (10) mindestens eines zweidimensionalen Bildes der Wirbelsäule eines Patienten;
b) Auswählen (40) mindestens eines dreidimensionalen rekonstruierten Bildes eines Wirbels aus einer vordefinierten Datenbank, wobei das genannte rekonstruierte Bild eine vorgegebene Übereinstimmung mit Wirbelbildern des zweidimensionalen Bildes besitzt;
c) Erstellen (30) eines dreidimensionalen Modells der Wirbelsäule durch Kombination (50) des rekonstruierten Bildes und des zweidimensionalen Bildes, um ein rekonstruiertes dreidimensionales Modell der Wirbelsäule zu erhalten;
d) Durchführen (60) einer sagittalen Gleichgewichtsanalyse des zweidimensionalen Bildes oder des dreidimensionalen Modells zur Berechnung von Wirbelsäulen-Becken-Parametern und Vergleichen der genannten Wirbelsäulen-Becken-Parameter mit vorgegebenen Bereichswerten;
e) Anbringen (70) dreidimensionaler simulierter Korrekturen an dem dreidimensionalen Modell der Wirbelsäule;
f) Berechnen (80) der korrigierten Wirbelsäulen-Becken-Parameter auf dem dreidimensionalen Modell der Wirbelsäule, das mit den simulierten Korrekturen korrigiert wurde, und Überprüfen, ob die korrigierten Wirbelsäulen-Becken-Parameter innerhalb der vorgegebenen Bereichswerte liegen,
g) im negativen Fall, Wiederholen der Schritte e) - f);
i) im positiven Fall, Erhalten (90) eines dreidimensionalen korrigierten Modells der Wirbelsäule basierend auf solchen dreidimensionalen simulierten Korrekturen, wobei die genannten dreidimensionalen simulierten Korrekturen das Einsetzen einer Vielzahl von virtuellen Schrauben in das dreidimensionale Modell der Wirbelsäule einschließen;
j) Berechnen (100) der Form der Stange als die mathematische Funktion, die sich am besten einer Kurve annähert, die durch die Vielzahl der virtuellen Schrauben des dreidimensionalen korrigierten Modells der korrigierten Wirbelsäule verläuft.

## Revendications

1. Procédé de calcul de la forme d'une tige d'un système de vis pédiculaires pour la spondylodèse, comprenant les étapes consistant à :
a) acquérir (10) au moins une image bidimensionnelle de la colonne vertébrale d'un patient ;
b) sélectionner (40) au moins une image tridimensionnelle reconstruite d'une vertèbre à partir d'une base de données prédéfinie, ladite image reconstruite ayant une correspondance prédéterminée avec les images de vertèbres de l'image bidimensionnelle ;
c) obtenir (30) un modèle tridimensionnel de la colonne vertébrale en combinant (50) l'image reconstruite et l'image bidimensionnelle pour obtenir un modèle tridimensionnel reconstruit de la colonne vertébrale ;
d) effectuer (60) une analyse d'équilibre sagittal de l'image bidimensionnelle ou du modèle tridimensionnel pour calculer des paramètres spino-pelviens et comparer lesdits paramètres spino-pelviens à des valeurs de plage prédéterminées ;
e) appliquer (70) des corrections tridimensionnelles simulées au modèle tridimensionnel de la colonne vertébrale ;
f) calculer (80) des paramètres spino-pelviens corrigés sur le modèle tridimensionnel de la colonne vertébrale corrigé à l'aide des corrections simulées et vérifier si au moins un sous-ensemble des paramètres spino-pelviens corrigés se situe dans les valeurs de plage prédéterminées,
g) dans le cas négatif, répéter les étapes e) et f) ;
i) dans le cas positif, obtenir (90) un modèle tridimensionnel corrigé de la colonne vertébrale sur la base de telles corrections tridimensionnelles simulées, lesdites corrections tridimensionnelles simulées comprenant l'insertion d'une pluralité de vis virtuelles dans le modèle tridimensionnel de la colonne vertébrale ;
j) calculer (100) la forme de la tige comme la fonction mathématique qui se rapproche le plus d'une courbe passant par la pluralité de vis virtuelles du modèle tridimensionnel corrigé de la colonne vertébrale corrigée.

2. Procédé de calcul de la forme d'une tige selon la revendication 1, où l'étape consistant à combiner (50) l'au moins une image reconstruite et l'image bidimensionnelle comprend l'application d'un algorithme de radiographie numérique reconstruite (DRR).

3. Procédé de calcul de la forme d'une tige selon les revendications 1 ou 2, où l'au moins une image bidimensionnelle comprend une image radiographique latérale et/ou antéro-postérieure.

4. Procédé de calcul de la forme d'une tige selon l'une quelconque des revendications précédentes, où l'étape consistant à sélectionner (40) est réalisée en sélectionnant (40) au moins une image tridimensionnelle reconstruite de chaque vertèbre à partir d'une base de données prédéfinie ou le procédé comprend une étape consistant à acquérir (20) une image tridimensionnelle d'au moins une vertèbre de la colonne vertébrale du patient et l'étape consistant à obtenir (30) un modèle tridimensionnel de la colonne vertébrale est réalisée en combinant pour chaque vertèbre l'image bidimensionnelle avec l'image reconstruite ou l'image tridimensionnelle.

5. Procédé de calcul de la forme d'une tige selon la revendication 4, où l'image tridimensionnelle de la colonne vertébrale comprend une image IRM ou TDM.

6. Procédé de calcul de la forme d'une tige selon l'une quelconque des revendications précédentes, où les paramètres spino-pelviens comprennent au moins l'un parmi : l'incidence pelvienne, la pente sacrée, la bascule pelvienne, la lordose lombaire, la cyphose thoracique, l'axe vertical sagittal.

7. Procédé de calcul de la forme d'une tige selon l'une quelconque des revendications précédentes, consistant en outre à ajouter des premiers facteurs de correction prédéterminés aux paramètres spino-pelviens corrigés, en fonction de la technique chirurgicale à appliquer et/ou de la mobilité et de la rigidité attendues de la colonne vertébrale, prenant ainsi en compte la déformation du matériau élastique de la tige.

8. Procédé de calcul de la forme d'une tige selon la revendication 7, consistant en outre à appliquer des seconds facteurs de correction prédéterminés aux paramètres spino-pelviens corrigés pour tenir compte de l'alignement du bassin et des membres inférieurs.

9. Système de calcul de la forme d'une tige d'un système de vis pédiculaires pour la spondylodèse comprenant un poste de travail (200) ayant un sous-système de traitement (210) agencé pour :
a) acquérir (10) au moins une image bidimensionnelle de la colonne vertébrale d'un patient ;
b) sélectionner (40) au moins une image tridimensionnelle reconstruite d'une vertèbre à partir d'une base de données prédéfinie, ladite image reconstruite ayant une correspondance prédéterminée avec les images de vertèbres de l'image bidimensionnelle ;
c) obtenir (30) un modèle tridimensionnel de la colonne vertébrale en combinant (50) l'image reconstruite et l'image bidimensionnelle pour obtenir un modèle tridimensionnel reconstruit de la colonne vertébrale ;
d) effectuer (60) une analyse d'équilibre sagittal de l'image bidimensionnelle ou du modèle tridimensionnel pour calculer des paramètres spino-pelviens et comparer lesdits paramètres spino-pelviens à des valeurs de plage prédéterminées ;
e) appliquer (70) des corrections tridimensionnelles simulées au modèle tridimensionnel de la colonne vertébrale ;
f) calculer (80) des paramètres spino-pelviens corrigés sur le modèle tridimensionnel de la colonne vertébrale corrigé à l'aide des corrections simulées et vérifier si les paramètres spino-pelviens corrigés se situent dans les valeurs de plage prédéterminées,
g) dans le cas négatif, répéter les étapes e) et f) ;
i) dans le cas positif, obtenir (90) un modèle tridimensionnel corrigé de la colonne vertébrale sur la base de telles corrections tridimensionnelles simulées, lesdites corrections tridimensionnelles simulées comprenant l'insertion d'une pluralité de vis virtuelles dans le modèle tridimensionnel de la colonne vertébrale ;
j) calculer (100) la forme de la tige comme la fonction mathématique qui se rapproche le plus d'une courbe passant par la pluralité de vis virtuelles du modèle tridimensionnel corrigé de la colonne vertébrale corrigée.
